# EUROPEAN PATENT APPLICATION

(11) **EP 1 553 394 A1**
(43) Date of publication of application: **13.07.2005**
(21) Application number: 03751391.8
(22) Date of filing: 09.10.2003
(51) Int. Cl.: G01N 1/10

(54) **SPUTUM-COLLECTING TOOL**

(30) Priority: 09.10.2002 JP 2002295809
(71) Applicant: Sunstar Inc., Takatsuki-shi, Osaka 569-1134 (JP)
(72) Inventor: EGUCHI, Toru, Takatsuki-shi, Osaka 569-1044 (JP); SUSI, Michael, Marlborough, MA 01752 (US); SULLIVAN, John, Atkinson, NH 03811 (US); HASLOB, Jason, Framingham, MA 01702 (US); KAHUTE, Trent, Charlestown, MA 02129 (US); KANE, William J., Sutton, MA 01590 (US)
(74) Representative: Smith, Norman Ian
(86) International application number: PCT/JP2003/012931
(87) International publication number: WO 2004/038382

(57) **Abstract**

An easy-to-gasp sputum collecting tool facilitating sputum collection work. The present sputum-collecting tool has a sputum-collecting device with a base part having a sputum-collecting portion for collecting sputum at one end and a handle portion at the other end, and a collection sample container for receiving the sputum-collecting device inside it. Grip faces positioned at both ends in a thickness direction of the handle portion are formed substantially flat, and a left/right width dimension of the handle portion is set larger than a left/right width dimension of the base portion.

## Description

### TECHNICAL FIELD

The present invention relates to a sputum-collecting tool and sputum-collecting tool kit which are used to collect sputum so that, for example, hepatitis C, diabetes, Alzheimer's disease, narcotics, AIDS, periodontal disease, dental caries (cavities), cancer of the oral cavity and the like can be easily discovered from sputum.

### BACKGROUND ART

For example, sputum-collecting tools constructed from a sputum-collecting device which holds filter paper used to absorb and collect sputum by means of a holding tool, and a cylindrical test sample container used to accommodate the abovementioned sputum-collecting device that has collected sputum, have been proposed as the abovementioned sputum-collecting tools. For instance, such a sputum-collecting tool is indicated in Japanese Patent Application Laid-Open No. 5-187976 (see Fig. 14).

### DISCLOSURE OF THE INVENTION

In the construction disclosed in Japanese Patent Application Laid-Open No. 5-187976, since the test sample container is cylindrical, the cover member that closes off the open end part of the test sample container is also naturally circular, and the handle part of the sputum-collecting device that is accommodated in the abovementioned test sample container by removing the cover member of the abovementioned test sample container has a circular disk form shape so that this handle part can be used as a cap that can close off the open end part of the test sample container. Accordingly, in cases where sputum is collected by grasping the handle part of the sputum-collecting device and causing the sputum-collecting device to contact the tongue, only some of the fingers can be caused to contact the circular arc form outer circumferential surface of the handle part, so that the circular arc form outer circumferential surface of the handle part tends not to fit the fingers. Accordingly, unless the handle part is grasped carefully, the fingers may tend to slip from the handle part, so that sputum collection is difficult. Furthermore, handling is similarly difficult when the sputum-collecting device is inserted into the test sample container.

In view of the conditions described above, it is an object of the present invention to provide a sputum-collecting tool that is easy to grip, so that sputum collection work can easily be performed.

In order to solve the abovementioned problems, the sputum-collecting tool of the present invention comprises a sputum-collecting device in which a handle part is disposed on one end of a base part which has a sputum-collecting part that collects sputum attached to the other end, and a collection sample container for accommodating this sputum-collecting device therein; furthermore, grip faces that are positioned on both ends of the abovementioned handle part in a thickness direction are formed substantially flat, and the left-right width dimension of the abovementioned handle part is set greater than the left-right width dimension of the abovementioned base part.

As a result of the grip faces of the handle part being formed substantially flat as described above, these grip faces easily fit the surfaces of the fingers; accordingly, the grip faces can easily be gripped and held by the fingers, so that there is no slipping of the fingers from the handle part, and sputum collection work can be quickly performed. Furthermore, since the left-right width dimension of the handle part is greater than the left-right width dimension of the base part, the tool has an action that can not only prevent accidental swallowing, but can also limit the length that is taken in, which is advantageous in terms of handling. Moreover, the present invention also offers the following advantage: namely, the contact area of the fingers with respect to the handle part is increased, so tat the tool can be held more stably.

Furthermore, recesses or protrusions, or both, may be formed in substantially the central portions of the abovementioned grip faces.

In cases where recesses are formed in the grip faces as described above, portions of the inside protrusions of the fingers can be inserted and caused to bite into these recesses, so that the fingers tend not to slip, thus allowing holding of the tool with good stability. Furthermore, in cases where protrusions are disposed on the abovementioned grip faces, these protrusions bite into the inside protrusions of the fingers so that the fingers tend not to slip. Moreover, in cases where both protrusions and recesses are disposed, the following advantage is similarly obtained: namely, portions of the inside protrusions of the fingers are inserted and bite into the recesses, and the protrusions bite into portions of the inside protrusions of the fingers, so that the fingers tend not to slip.

Furthermore, by disposing a stopper on the base part end portion of the abovementioned handle part which is used to prevent the fingers grasping this handle part from moving toward the base part by contacting the fingers, it is easily possible to hold the handle part in the gripped position with the fingers, so that the tool can be held much more stably. The abovementioned stopper part may have any shape, as long as this stopper part is a protrusion which protrudes in a direction that is substantially perpendicular to the grip faces.

Furthermore, a fiber bundle formed by bundling a plurality of fibers for absorbing the sputum collected by the abovementioned sputum-collecting part is disposed on the abovementioned base part, and an intermediate portion of this fiber bundle is colored with a coloring agent that can be dispersed by the sputum.

By utilizing the capillary phenomenon created by the numerous fibers as described above, it is immediately possible to grasp the amount of sputum that has been collected, as a result of the sputum being quickly drawn up and quickly dispersed by the coloring agent, so that a sputum-collecting tool that is advantageous in terms of use can be constructed.

Furthermore, the abovementioned collection sample container is constructed from a container main body which is equipped at one end thereof with a removal piece that forms a projection protruding to the outside, and that can be cut away to allow removal of an internal accumulated liquid, and a freely detachable closing cap for closing off an opening part formed in the other end of this container main body.

Accordingly, the liquid accumulated inside the container can be quickly removed to the outside by cutting away the removal piece.

Furthermore, by disposing a flange part for placing the abovementioned collection sample container in a tightly closed state by engaging with the inner surface of this collection sample container when the insertion of the sputum-collecting device into this collection sample container is completed between the handle part and base part of the abovementioned sputum-collecting device, it is possible to engage the flange part with the inside surface of the collection sample container so that the interior of the collection sample container is placed in a tightly closed state merely by inserting the sputum-collecting device into the collection sample container, so that there is no need for any special sealing member, which is advantageous in terms of assembly and cost.

Furthermore, since at least a portion of the handle part of the abovementioned sputum-collecting device is covered by the container main body of the abovementioned collection sample container in a state in which the insertion of this sputum-collecting device into the abovementioned collection sample container is completed, the unexpected slipping out of the sputum-collecting device from the collection sample container as a result of some other object contacting the sputum-collecting device can be avoided.

Furthermore, if the abovementioned collection sample container is made self-standing when sputum is collected by the abovementioned sputum-collecting device by disposing a leg part for making this collection sample container self-stand on the removal piece of the abovementioned collection sample container, the collection of sputum can be performed while operating the sputum-collecting device with both hands. The operation can be completed merely by inserting the sputum-collecting device that has collected the abovementioned sputum into the collection sample container in a self-standing state. Besides being detachably attached to the removal piece of the collection sample container by engagement or anchoring, it would also be possible to devise the abovementioned leg part so that this leg part can be fastened to the removal piece of the collection sample container and removed by being cut away (or twisted off); alternatively, it would also be possible to attach the leg part to the removal piece of the collection sample container so that this leg part can be folded double and thus folded into a small size when not needed.

Furthermore, if the handle part of the abovementioned sputum collecting device is disposed in the base part of the abovementioned sputum collecting device so that the abovementioned handle part can be separated from the abovementioned base part as the abovementioned handle part is pulled out in order to remove the abovementioned sputum collecting device from the abovementioned collection sample container in a state in which the abovementioned sputum collecting device is inserted into and accommodated in the abovementioned collection sample container, then even if an attempt is made to pull the sputum-collecting device out of the collection sample container after sputum collection has been completed and the sputum-collecting device has been inserted into the collection sample container, only the handle part is separated from the base part, so that the base part maintains an accommodated state in which the base part is inserted into the collection sample container. Accordingly, re-insertion of the sputum-collecting device with adhering preservative liquid or the like into the mouth after this sputum-collecting device has been pulled out of the collection sample container, and the entry of foreign matter or the like into the collection sample container after the sputum-collecting device has been pulled out of the collection sample container, can be securely prevented.

Furthermore, if a construction is used in which the abovementioned collection sample container comprises a bottomed-cylindrical lower part which accommodates the base part of the abovementioned sputum-collecting device, and an upper part which is integrally connected with the upper end portion of this lower part, and which is constructed with a size so as to cover at least a portion of the handle part of this sputum-collecting device from the outside, and a cut part is provided which allows the abovementioned lower part and upper part to be cut apart in the connecting portion of these parts, then, if the upper part which has a greater left-right width than the lower part is cut away via this cut part at the time of mounting in the centrifuging tube of a centrifugal separator, collisions that are caused by the upper part with a large left-right width when the centrifuging tubes are lined up on the tube rack following mounting in each centrifuging tube can be prevented. In this case, an even smaller size can be obtained removing the handle part from the base part of the sputum-collecting device, so that this is desirable. Furthermore, the sputum-collecting tool as a whole can be reduced in size if the upper part is cut away not onl6 in the case of mounting in the abovementioned centrifuging tubes, but also in the case of shipping by mail as described above, so that such cutting away is desirable. In this case as well, it is even more desirable if the handle part is removed from the base part of the sputum-collecting device. Moreover, the upper part can simply be twisted off by means of the simple construction of providing a cut part.

Furthermore, if the abovementioned sputum-collecting part is constructed from polyurethane, the adsorption of protein can be suppressed, and the total cost can be reduced, while eliminating the need for operations such as (for example) impregnating a non-woven fabric with bovine albumin or performing a water impregnation treatment.

Moreover, if the abovementioned sputum-collecting part is constructed from cellulose acetate, the adsorption of protein can be suppressed while eliminating the need for operations such as (for example) impregnating a non-woven fabric with bovine albumin or performing a water impregnation treatment, in the same manner as in the case of the abovementioned polyurethane. In this case, furthermore, not only can large amounts of sputum be absorbed, but there is no swelling accompanying sputum absorption as there is in the case of polyurethane, so that insertion into the collection sample container can be smoothly accomplished.

Moreover, if the thickness of the abovementioned collection sample container is set at 10 mm or less, this container can be sent as a fixed type postal item of ordinary mail as stipulated in Japan, which is a point that allows for easy utilization.

A sputum-collecting tool kit with high commercial value can be obtained by constructing a sputum-collecting tool kit which comprises a box form casing main body accommodating a sputum-collecting tool which comprises a sputum-collecting device in which a handle part is disposed on one end of a base part which has a sputum-collecting part that collects sputum attached to the other end, and a collection sample container for accommodating this sputum-collecting device therein, and which is characterized in that grip faces that are positioned on both ends of the abovementioned handle part in a thickness direction are formed substantially flat, and the left-right width dimension of this handle part is set greater than the left-right width dimension of the abovementioned base part, and a cover body which is attached to the abovementioned casing main body so that this cover body can be freely opened and closed by swinging, in order to close off the open side of the abovementioned casing main body in which the sputum-collecting tool is accommodated.

Moreover, if a holding part for holding the abovementioned collection sample container in an upright attitude is provided in the abovementioned casing main body, the need to hold the collection sample container in one hand is eliminated, so that the device is superior in terms of operating characteristics, and so that the sputum-collecting tool can be inserted into the collection sample container inside the casing main body.

Furthermore, if a diagram explaining the use of the tool is printed on the inside surface of the abovementioned cover body, or a printed diagram explaining use is attached to this inside surface, the method of use of the tool can be immediately understood merely by placing the cover body in an open state, e. g., without an need for opening separately attached instructions. Moreover, the sputum-collecting tool and collection sample container can be quickly operated while viewing this diagram explaining use, so that the commercial value of the tool can be greatly increased.

Moreover, if an accommodating part that can accommodate an enveloped used for mailing is disposed on the abovementioned cover body, such an enveloped used for mailing can be provided in the cover body in an orderly state, so that the tool is much more advantageous in terms of handling.

### BRIEF DESCRIPTION OF THE DRAWINGS

Fig. 1 is a perspective view of the sputum-collecting tool kit with the cover body in an open state;
Fig. 2 is an exploded perspective view of the sputum-collecting tool kit;

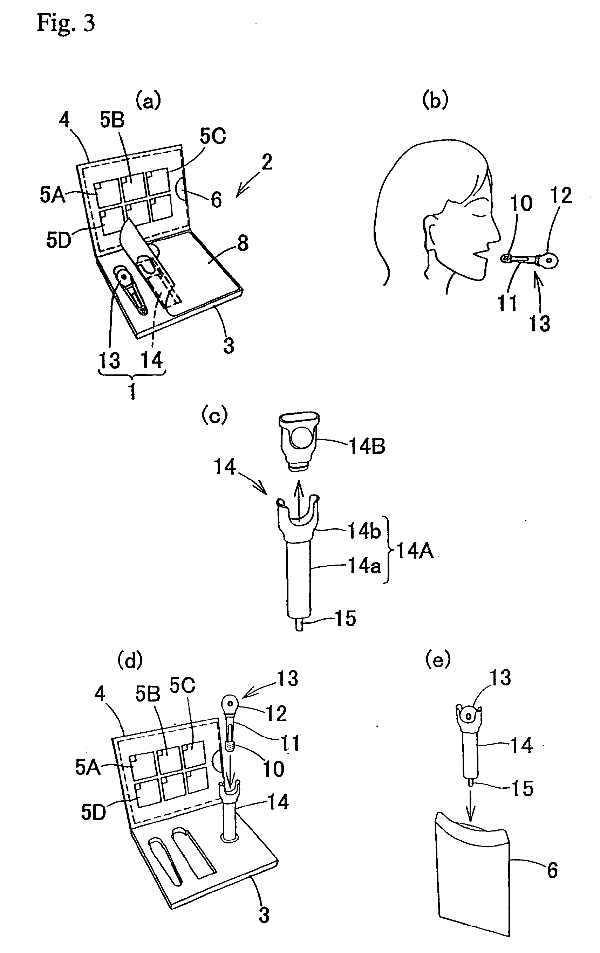

Fig. 3 shows explanatory diagrams that illustrate the method of use of the sputum-collecting tool kit, with Fig. 3 (a) showing the state immediately prior to the removal of the sputum-collecting tool following the opening of the cover body, Fig. 3 (b) showing the state immediately prior to the insertion of the sputum-collecting device into the mouth, Fig. 3 (c) showing a state in which the cap of the collection sample container is removed, Fig. 3 (d) showing the state immediately prior to the insertion of the sputum-collecting device that has collected sputum into the collection sample container, and Fig. 3 (e) showing the state immediately prior to the insertion of the integrated collection sample container into the envelope following the insertion of the sputum-collecting device into the collection sample container;
Fig. 4 shows the sputum-collecting device, with Fig. 4 (a) showing a perspective view of this sputum-collecting device, Fig. 4 (b) showing a partially sectional side view of the upper part of the same, Fig. 4 (c) showing a plan view, and Fig. 4 (d) showing a sectional view along line I-I in Fig. 4 (a);
Fig. 5 shows the collection sample container, with Fig. 5 (a) showing a perspective view of the collection sample container, Fig. 5 (b) showing a plan view of the collection sample container in a state in which the cap has been removed, and Fig. 5 (c) showing a sectional view along line II-II in Fig. 5 (a);
Fig. 6 is a perspective view which shows the state immediately prior to the insertion of the sputum-collecting device that has collected sputum into the collection sample container;
Fig. 7 is a perspective view of the collection sample container into which the sputum-collecting device has been inserted and integrated;
Fig. 8 is a longitudinal sectional view of the collection sample container into which the sputum-collecting device has been inserted and integrated;
Fig. 9 shows longitudinal sectional views of portions of the removal piece of the collection sample container, with Fig. 9 (a) showing the state immediately prior to the twisting off of the removal piece, and Fig. 9 (b) showing a state in which the removal piece has been twisted off;
Figs. 10 (a) through 10 (e) show front views and side views of other sputum-collecting devices;

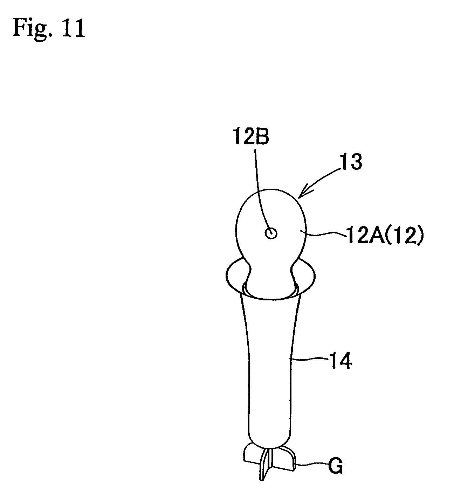

Fig. 11 is a perspective view showing a state in which the collection sample container into which the sputum-collecting device has been inserted is held in an upright attitude;
Fig. 12 is an exploded perspective view showing another sputum-collecting device;
Fig. 13 is a side view of the sputum-collecting device shown in Fig. 12;
Fig. 14 is a perspective view showing another collection sample container in a state in which the cap has been removed;
Fig. 15 is a longitudinal sectional front view of the collection sample container shown in Fig. 14;
Fig. 16 is a longitudinal sectional front view showing a state in which the sputum-collecting device shown in Fig. 12 has been inserted into the collection sample container shown in Fig. 14; and
Fig. 17 is a longitudinal sectional front view showing a state in which the handle part and upper part have been removed in the collection sample container shown in Fig. 16.

### BEST MODE FOR CARRYING OUT THE INVENTION

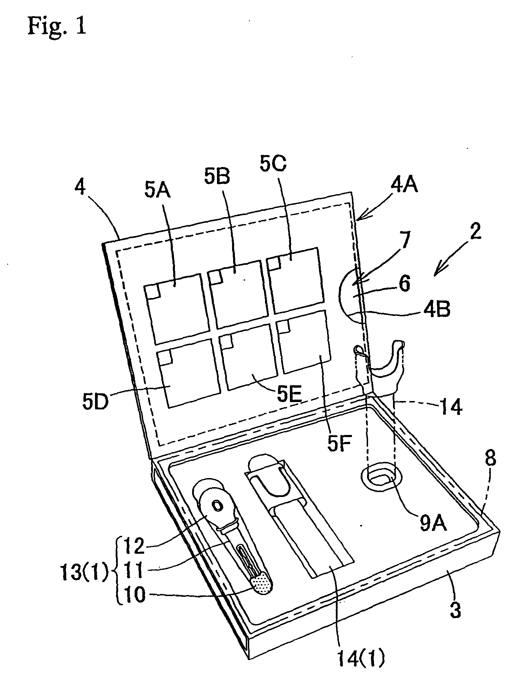

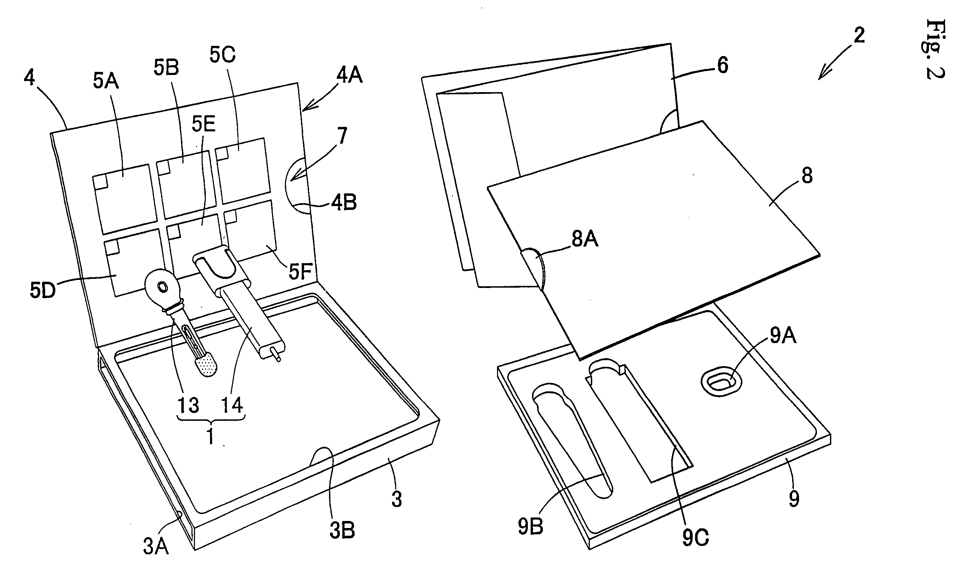

A sputum-collecting tool kit 2 accommodating the sputum-collecting tool 1 of the present invention is shown in Fig. 1, and an exploded perspective view of the parts constituting this sputum-collecting tool kit 2 is shown in Fig. 2. The abovementioned sputum-collecting tool kit 2 comprises a substantially rectangular box-form paper (or plastic) casing main body 3 which accommodates the abovementioned sputum-collecting tool 1, and a paper (or plastic) cover body 4 which is attached to one of the two long sides of the casing main body 3 in order to close off the open side (upper side) of this casing main body 3, so that this cover body 4 is free to open and close by swinging. The abovementioned casing main body 3 is constructed so that the upper surface and one of the four side surfaces are open, so that a substantially rectangular tray 9 made of plastic and molded by vacuum molding can be inserted from the side-surface opening part 3A, and so that the upper surface of this inserted tray 9 can be exposed via the upper-surface opening part 3B for the tray 9. As is shown in Fig. 2, recesses 9B and 9C which can accommodate a sputum-collecting device 13 and collection sample container 14 that constitute the abovementioned sputum-collecting tool 1 are disposed in the abovementioned tray 9, and the system is devised so that the sputum-collecting device 13 and collection sample container 14 cannot move irregularly when accommodated in these recesses 9B and 9C. However, it would also be possible to work the present invention by disposing anchoring fittings that respectively anchor and hold the sputum-collecting device 13 and collection sample container 14 in the casing main body 3 instead of the recesses 9B and 9C, and the means used to fix the positions of the sputum-collecting device 13 and collection sample container 14 in place with respect to the casing main body 3 can be freely altered. Depending on the case, furthermore, such means used to fix the positions of the abovementioned sputum-collecting device 13 and collection sample container 14 may be omitted; for example, such parts may be combined as a bag or the like.

A diagram explaining use is printed on the inside surface (undersurface) of the abovementioned cover body 4. As is shown in Figs. 1 and 2, six explanatory diagrams 5A through 5F can be visually recognized by placing the cover body 4 in an open state. Accordingly, the following advantage is obtained: namely, not only can the method of use be checked immediately merely by placing the cover body 4 in an open state, but sputum collection can be performed without error while viewing the explanatory diagrams 5A through 5F during use as well. However, it would also be possible to work the invention without integrating the cover body 4 and casing main body 3, e. g., by separately manufacturing the casing main body 3 and cover body 4, printing a diagram explaining use on the abovementioned cover body 4, constructing this cover body 4 so the cover body 4 is self-standing, superimposing the casing main body 3 and cover body 4, and achieving integration by packaging using packaging paper or the like. Here, furthermore, an embodiment is described in which as diagram explaining use is directly printed on the inside surface of the cover body 4; however, it would also be possible to bond a paper or plastic sheet member or the like on which a diagram explaining used is printed to the inside surface of the cover body 4, or to fasten such a member in place using an anchoring mechanism or the like. Furthermore, it would also be possible to construct the system so that the abovementioned cover body 4 can be held at a specified angle (e. g., an arbitrary angle in the range of 90 degrees to less than 180 degrees) with respect to the upper surface of the casing main body 3, or to construct the system so that the system can be used in a state in which the cover body is opened 180 degrees.

Furthermore, an accommodating part 7 which can accommodate a mailing envelope 6 used for the postal shipping of the abovementioned sputum-collecting tool 1 that has collected sputum is disposed on the abovementioned cover body 4. Namely, an opening part 4A is formed in the right side of the cover body 4 in' Figs. 1 and 2, and the system is devised so that this enveloped 6 can be inserted and accommodate inside the cover body 4 via this opening part 4A. A circular arc form cut-out 4A is formed in substantially the central portion (with respect to the outer edge direction) in the inside portion of the right-side outer edge part of the cover body 4, so that the following advantage is obtained: namely, the abovementioned envelope 6 that has been accommodated can easily be removed via the cut-out 4B. However, the cut-out 4B may also be omitted in the working of the present invention. The abovementioned enveloped 6 has a size that allows postal shipping as a fixed type postal item among first class mail constituting ordinary mail according to Japanese postal regulations. However, some other size may also be used. The member 8 is a sheet form (or thin film form) paper (or plastic) member 8 which is bonded to the upper surface of the casing main body 3 in order to achieve tight hygienic sealing of the sputum-collecting tool 1 accommodated in the casing main body 3 shown in Figs. 1 and 2, and the system is devised so that the member 8 can easily be peeled away by grasping and pulling an extension part 8A on the left side.

As is shown in Fig. 1, Figs. 4 (a) through 4 (d) and Figs. 5 (a) through 5 (c), the abovementioned sputum-collecting tool 1 comprises a sputum-collecting device 13 made of a synthetic resin in which a handle part 12 is disposed on one end of a base part 11 which has a sputum-collecting part 10 with a substantially spherical tip end surface (lower end surface) consisting of polyurethane or cellulose acetate (that absorbs and collects sputum) attached to the other end, and a collection sample container 14 made of a synthetic resin that is used for the insertion and accommodation of this sputum-collecting device 13.

A low protein bonding type polyurethane whose surface has been treated with a surfactant or macromolecular compound shows a lower protein absorption than a general polyurethane, and is therefore desirable from the standpoint of sputum testing as the abovementioned polyurethane; however, a general polyurethane may also be used. The abovementioned polyurethane swells when sputum is absorbed. However, the abovementioned cellulose acetate not only absorbs a larger amount of sputum than the abovementioned polyurethane, but also shows no swelling when sputum is absorbed; accordingly, this cellulose acetate offers the advantages of allowing smooth insertion of the sputum-collecting part 10 into the collection sample container 14 following sputum absorption, and allowing an increase in the sputum recovery rate into the collection sample container 14, so that such cellulose acetate is desirable. For example, Transorb manufactured by Filtrona Richmond Co. may be cited as an example of the abovementioned cellulose acetate resin.

Furthermore, the abovementioned handle part 12 has a substantially circular shape as seen in a front view; moreover, the diameter of this handle part 12 is set at a dimension that is roughly twice the left-right width dimension of the base part 11, and the grip faces 12A, 12A that are positioned on both ends in the thickness direction are formed substantially flat, so that the handle part 12 is easy to grip. Moreover, the thickness of the abovementioned handle part 12 is greater than the thickness of the base part 11; however, this handle part 12 may also have substantially the same thickness as the base part 11. Furthermore, substantially elliptical recesses 12B are formed in substantially the central portions of the respective grip faces 12A, 12B, so that the fingers tend not to slip from the handle part 12.

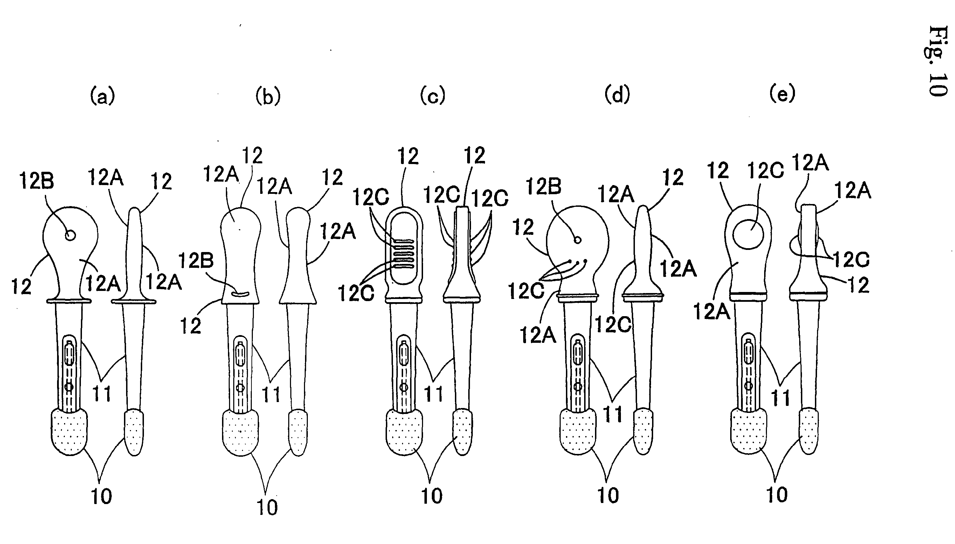

Substantially elliptical recesses 12B are formed in the abovementioned handle part 12; however, these may also be substantially circular recesses 12B that are formed in substantially the central portion of the handle part 12 as shown in Fig. 10 (a), substantially crescent shaped (circular arc form) recesses 12B that are formed in positions separated from the central portion of the handle part 12 as shown in Fig. 10 (b), five protrusions 12C that are long in the lateral direction, which are formed as protrusions at specified intervals in the longitudinal direction (vertical direction in the figures) of the handle part 12 as shown in Fig. 10 (c), circular recesses 12B that are formed in substantially the central portion of the handle part 12 and three circular protrusions 12C that are formed as protrusions at specified intervals from these recesses 12B on substantially the same curvature radius as shown in Fig. 10 (d), or protrusions 12C that are formed as protrusions in a range extending over substantially all of the central portion of the handle part 12 as shown in Fig. 10 (e). The shapes, sizes and numbers of the protrusions or recesses (or both) that are formed in the handle part 12 in order to prevent slipping of the fingers are not limited to the examples shown in the figures. Furthermore, the shape of the abovementioned handle part 12 as seen in a front view may also be substantially elliptical or the like as seen in Figs. 10 (b), 10 (c) and 10 (e). Furthermore, the grip faces 12A, 12A of the handle part 12 may have gently curved surfaces that gradually move inward approaching the central portions as shown in Fig. 10 (b). Alternatively, as is shown in Fig. 10 (d), these grip faces 12A, 12A may have gently curved surfaces that gradually protrude outward toward approaching the central portions. Cases in which the grip faces 12A, 12A are formed as substantially flat surfaces are also included in cases in which the grip faces are constructed as such gently curved surfaces.

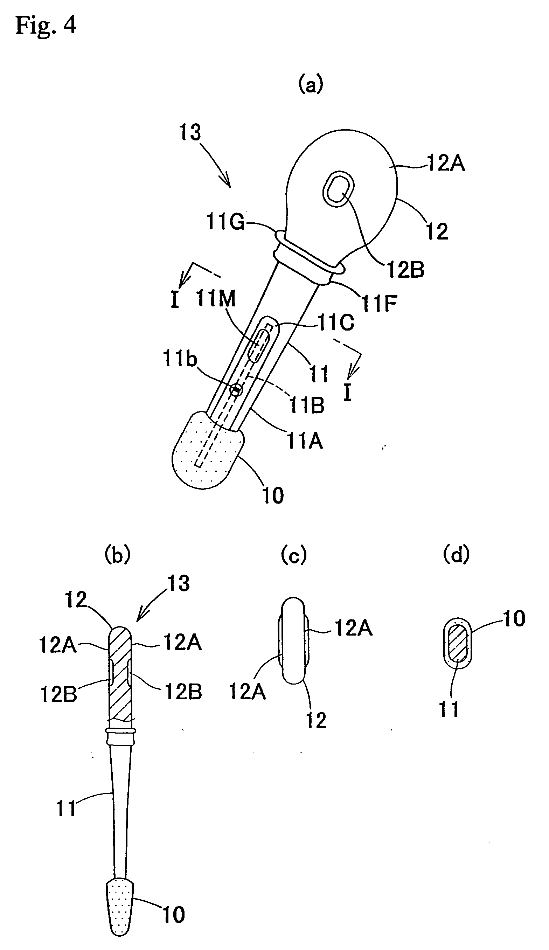

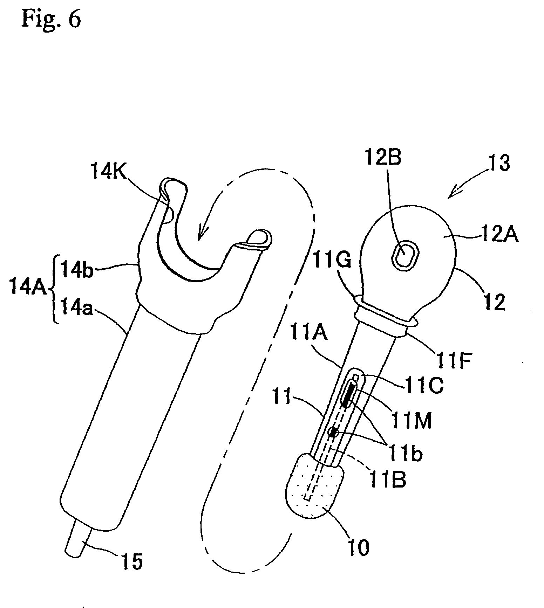

As is shown in Figs. 4 (a) through 4 (d), the abovementioned base part 11 comprises a base main body 11A with a substantially oval (or rectangular) cross-sectional shape which has the abovementioned sputum-collecting part 10 fit over one end, and in which the abovementioned handle part 12 is integrally formed on the other end, a fiber bundle 11B (formed by bundling numerous fibers) which is accommodated in this base main body 11A in order to suck up sputum collected by the sputum-collecting part 10 utilizing the capillary phenomenon, and a cover member 11C equipped with a transparent window 11M which is transparent so that the amount of sputum that is absorbed can be recognized by the upward movement of the coloring agent 11b together with the movement of the sputum (as shown for example in Fig. 6) as a result of this coloring agent 11b (with which an intermediate portion of the abovementioned fiber bundle 11B is colored) being dispersed by the absorbed sputum. However, this base part 11 may have some other construction.

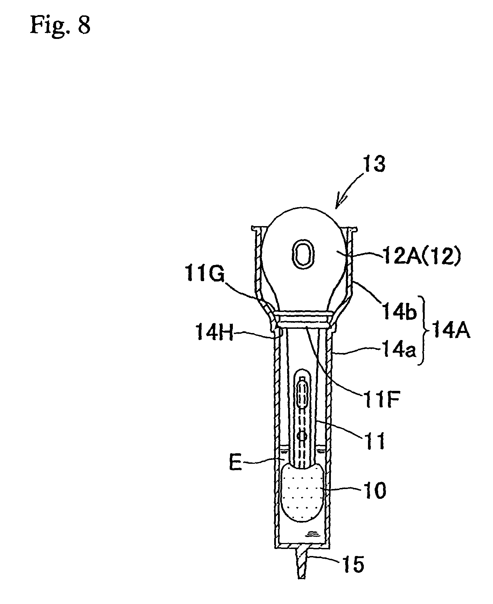

An annular flange part 11F which is used to engage with the inside surface of the collection sample container 14 and place this container in a tightly closed state when the insertion of the of the sputum-collecting device 13 into the abovementioned collection sample container 14 is completed is formed on the end portion of the abovementioned base main part 11A located on the side of the handle part 12, and as is shown in Fig. 8, the interior of the collection sample container 14 can be placed in a tightly closed state by the internal engagement of the flange part 11F with an annular engaging part 14H formed on the inside wall of the collection sample container 14 merely by inserting the sputum-collecting device 13 into the collection sample container 14. Furthermore, an annular protrusion 11G which is formed as a protrusion is formed as a stopper part that protrudes further outward than the flange part 11F, and stops the movement of the fingers grasping the handle part 12 toward he base part 11 by contacting these fingers, on the end portion of the abovementioned flange part 11F that is located on the side of the handle part 12. However, it would also be possible to form a plurality of protrusions as protrusions at appropriate intervals along the circumferential edge, or to work the invention with these protrusions omitted.

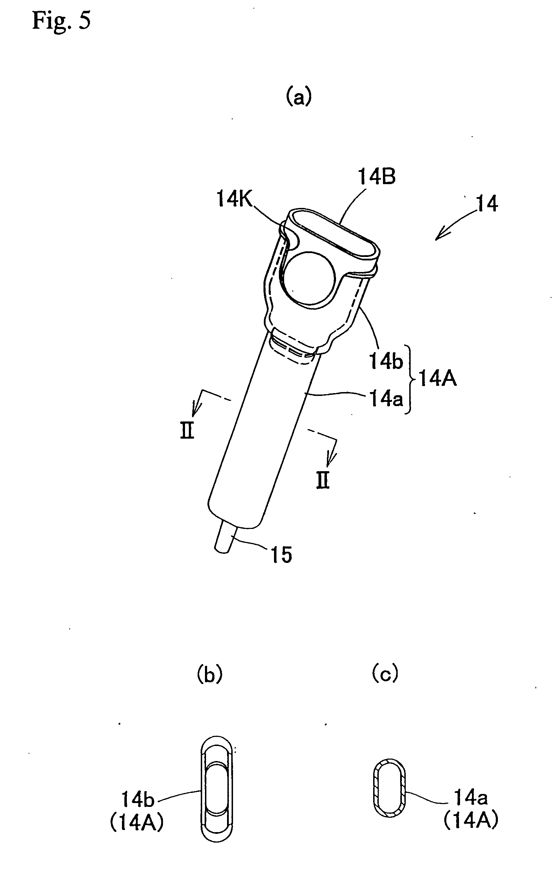

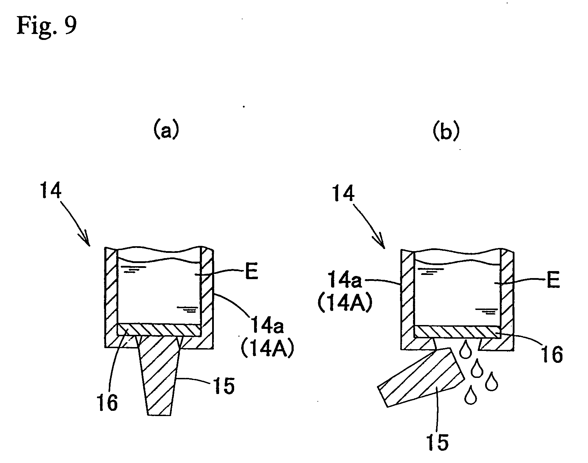

As is shown in Fig. 5 (a) through 5 (c) and Fig. 9 (a), the abovementioned collection sample container 14 contains a specified amount of accumulated preservative liquid E, and comprises a container main body 14A which has a substantially oval cross-sectional shape that allows the insertion of the abovementioned sputum-collecting device 13, and a cap 14B for closing off the upper end opening part of this container main body 14A. However, the present invention may also be worked by mounting a film inside the container main body 14A so that no accumulated preservative liquid E flows out to the outside. Furthermore, if the abovementioned film is constructed so that this film is ruptured when the sputum-collecting device 13 is inserted, this film can be used in a convenient manner.

The lower part 14a of the abovementioned container main body 14A is formed with a substantially oval cross-sectional shape that allows the insertion of the base part 11 of the abovementioned sputum-collecting device 13, and the upper part 14b is formed with a substantially circular cross-sectional shape which is larger than that of the abovementioned lower part 14a, and which allows the insertion of the handle part 12 of the abovementioned sputum-collecting device 13; furthermore, respective U-shaped cut-outs 14K are formed in both surfaces of the container main body 14A in the thickness direction. However, these cut-outs 14K may also be omitted. As a result of both the left and right sides of the handle part 12 of the sputum-collecting device 13 being covered by the abovementioned upper part 14b, other objects can be prevented from contacting the handle part 12.

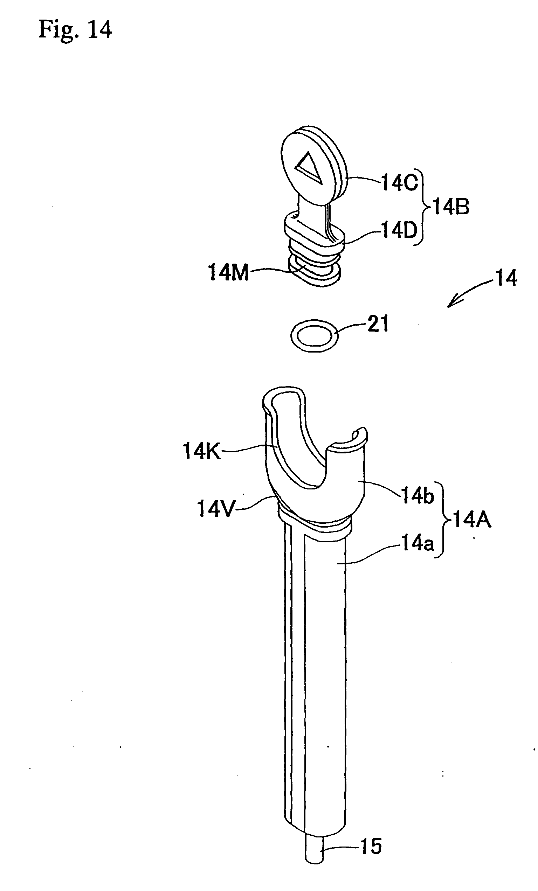

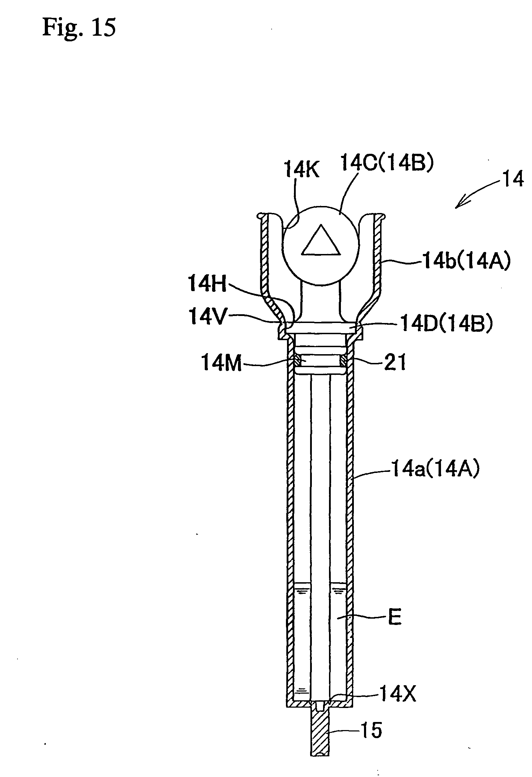

As is shown in Figs. 9 (a) and 9 (b), a round rod form (prism form or other form) removal piece 15 decreasing in diameter in the downward direction, which forms a projection that protrudes downward and which makes it possible to remove the abovementioned preservative liquid E (constituting an internal storage liquid) together with the sputum by being twisted off so that this removal piece 15 is cut away, is disposed on the lower end of the abovementioned collection sample container 14. This removal piece 15 may be formed as an integral part of the collection sample container 14, or a separate part may be attached to the collection sample container 14 by welding or the like. Moreover, the abovementioned removal piece 15 may also be devised so that this removal piece can be utilized as a leg that can make the collection sample container 14 self-standing by causing the abovementioned removal piece 15 to punch into the abovementioned tray 9 as shown in Fig. 3 (d). The symbol 16 in Figs. 9 (a) and 9 (b) indicates a filter, and this filter is devised so that dust or the like that has inadvertently been allowed to enter the collection sample container 14 does not exit the device together with the sputum; in some cases, however, this filter may be omitted. The present invention may also be embodied with this filter 16 disposed in the collection sample container 14 shown in Figs. 14 and 15.

Next, the method of use of the abovementioned sputum-collecting tool kit 2 will be described. First, after the cover body 4 is placed in closed state as shown in Fig. 3 (a), the member 8 is peeled away, and the sputum-collecting device 13 is removed from the casing main body 3. Next, after the handle part 12 of the sputum-collecting device 13 is grasped in order to perform sputum collection while viewing the explanatory diagrams of the cover body 4, the sputum-collecting part 10 is placed in the mouth, and sputum is adsorbed on this sputum-collecting part 10 as shown in Fig. 3 (b). During the collection of sputum by this sputum-collecting part 10, the collection sample container 14 is removed from the casing main body 3, and, after the cap 14B is removed from the collection sample container 14 as shown in Fig. 3 (c), an upright attitude (self-standing attitude) is maintained by inserting the removal piece 15 of the lower part 14a of the container main body 14A into a specified location of the tray 9 as shown in Fig. 3 (d). Furthermore, a recess 9A is formed as a holding part for holding the collection sample container 14 in an upright attitude in the location of the abovementioned tray 9 where the removal piece 15 is inserted as shown in Figs. 1 and 2, and the system is devised so that the abovementioned upright attitude can be maintained with good stability by inserting and positioning the lower end portion of the lower part 14a of the container main body 14A in this recess 9A; however, this recess 9A may also be omitted. It is judged by the condition of the coloring agent 11b whether or not the amount of sputum collected in the abovementioned sputum-collecting part 10 has exceeded the abovementioned specified amount. In cases where the coloring agent 11b can be seen through the window 11M as shown in Fig. 6, it is judged that the specified amount has been reached, the sputum-collecting device 13 is inserted from above into the collection sample container 14 set in the abovementioned self-standing attitude, and the sputum collection work is ended as shown in Fig. 3 (d). The collection sample container 14 into which this sputum-collecting device 13 has been inserted is removed from the casing main body 13, the collection sample container 14 is inserted into the envelope 6 as shown in Fig. 3 (e), and is sent by mail to a specified inspection organ where various types of inspections are performed, and the inspection results are then sent back.

In the case of a sputum-collecting tool kit 2 that is not equipped with the abovementioned casing main body 3, the present invention may also be worked by providing a leg part G with a cruciform shape that can achieve a self-standing attitude on the lower end of the collection sample container 14 (the shape is different from that shown in Fig. 5; however, this part may also have the same shape as that shown in Fig. 5) (a shape such as a circular disk form shape, square plate form shape or the like may be used as long as this shape can achieve a self-standing attitude) in a freely detachable manner or so that this part can easily be removed.

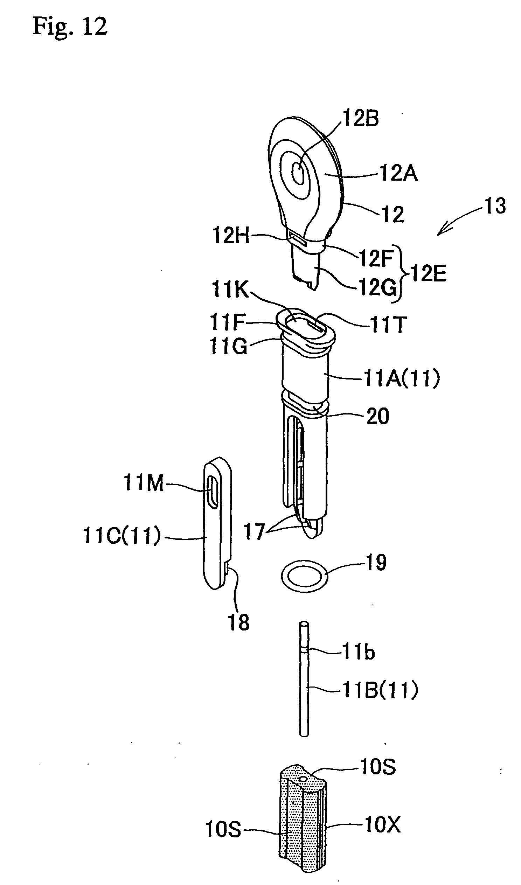

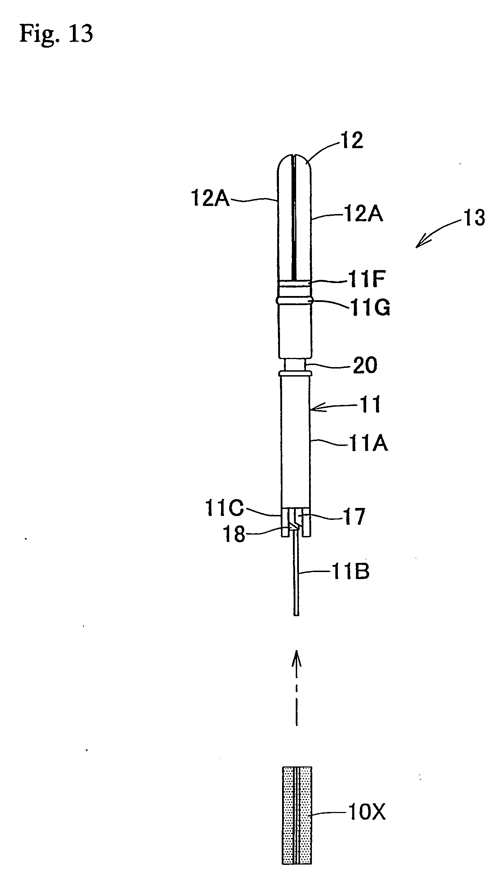

Figs. 12 and 13 show a sputum-collecting device 13 with a different construction, and Figs. 14 and 15 show a collection sample container 14 with a different construction. Here, a sputum-collecting tool kit 2 is constructed by accommodating such a sputum-collecting device 13 and collection sample container 14 in the casing main body 3 shown in Fig. 1, and this kit is used according to the method of use described below. Furthermore, in Figs. 12 through 16, parts that are not particularly described in the figures are the same as parts in Figs. 1 through 9.

In the sputum-collecting device 13 shown in Figs. 12 and 13, a sputum-collecting part 10X which has substantially the shape of a rectangular solid (consisting of a material with low protein absorption such as polyurethane or cellulose acetate in the same manner as described above), which has recesses 10S formed over the entire region in the longitudinal direction (vertical direction) in an intermediate part in the left-right lateral direction in both surfaces in the thickness direction, and which is used to absorb and collect sputum, is attached to one end, and a base part 11 to which a handle part 12 in which the abovementioned recesses 12B are formed in stages so that these recesses 12B increase in size in stages toward the grip faces 12A (two stages are shown in the figures; however, three or more stages may also be used) is detachably attached is disposed on the other end.

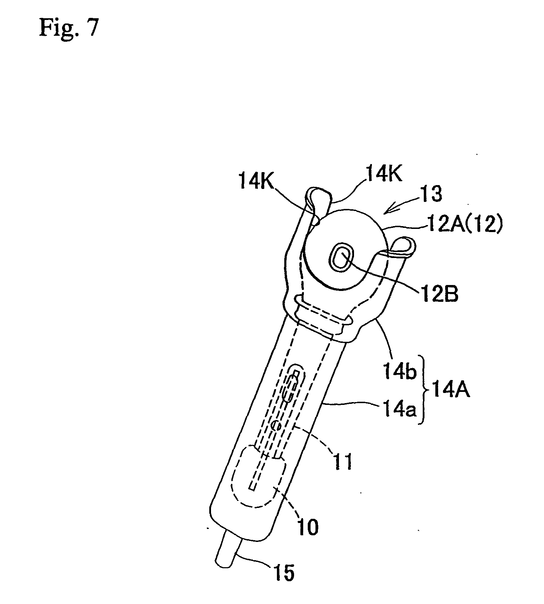

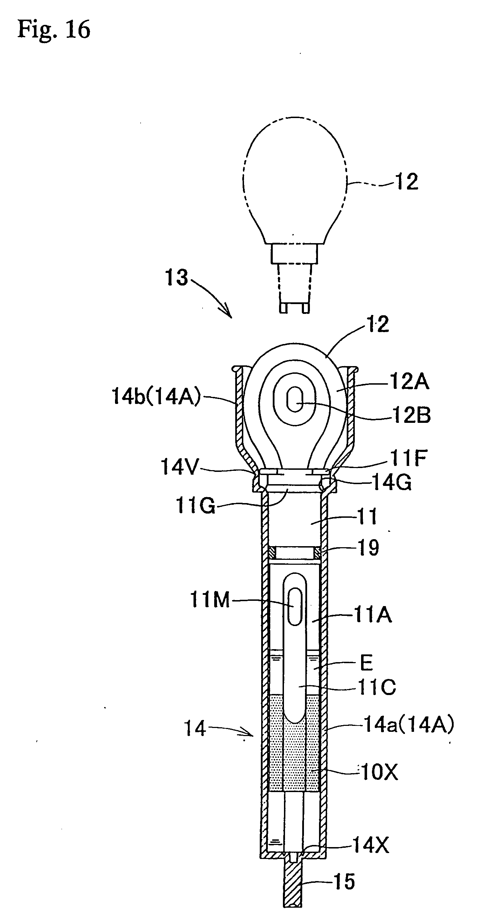

The abovementioned base part 11 comprises a base main body 11A in which an opening part 11K that allows the insertion of a skirt part 12E that is extended downward from the lower end of the abovementioned handle part 12 is formed on the upper end, a fiber bundle 11B (the same entity as that shown in Fig. 6; formed by bundling numerous fibers) which is accommodated in this base main body 11A in order to suck up sputum collected by the sputum-collecting part 10X utilizing the capillary phenomenon, and a cover member 11C equipped with a transparent window 11M which is transparent so that the amount of sputum that is absorbed can be recognized by the upward movement of the coloring agent 11b together with the movement of the sputum (as shown for example in Fig. 6) as a result of this coloring agent 11b (with which an intermediate portion of the abovementioned fiber bundle 11B is colored) being dispersed by the absorbed sputum. A flange part 11F which protrudes outward in the left-right direction from another position is formed on the upper end on which the opening part 11K of the abovementioned base main body 11A is formed, and a protrusion 11G which is used to perform positioning of the sputum-collecting device 13 by contacting the annular corner wall surface 14G that is formed on the upper end of the lower part 14a of the collection sample container 14 when the sputum-collecting device 13 is inserted into the collection sample container 14 as shown in Fig. 16 is formed on the portion directly beneath the abovementioned flange part 11F. Furthermore, pairs of left and right anchoring claws 17, 17 and 18, 18 that protrude to the inside of the base main body 11A are integrally formed on the inside surface of the abovementioned cover member 11C on the side of the sputum-collecting part 10X and the inside surface of the abovementioned base main body 11A on the side of the sputum-collecting part 10X, thus making it possible to maintain an anchoring effect that prevents the sputum-collecting part 10X from slipping off of the cover member 11C or base main body 11A by causing the anchoring claws 17, 17 and 18, 18 (only one of the anchoring claws 18 is shown in the figures) to bite into the abovementioned sputum-collecting part 10X in a state in which the abovementioned sputum-collecting part 10X is clamped between the cover member 11C and base main body 11A. Furthermore, an annular groove 20 that can hold ring-form packing 19 is formed in the vicinity of the central portion of the abovementioned base main body 11A in the longitudinal direction (vertical direction in the figures), so that the following advantage can be obtained: namely, if packing 19 is disposed in the base main body 11A, the leakage of the preservative liquid E or the like in a state in which the sputum-collecting device 13 that has collected sputum is inserted into the collection sample container 14 as shown in Fig. 16 can be more securely prevented than in a construction in which tight closing is accomplished by the engagement shown in Fig. 7. The shape, size and number of the abovementioned anchoring claws 17 and 18 are not limited to those shown in the figures.

The abovementioned skirt part 12E comprises an engaging part 12F which has a substantially oval external shape that allows internal engagement with the abovementioned substantially oval opening part 11K, and a guide part 12G having an external shape with smaller dimensions than those of the opening part 11K at the lower end of this engaging part 12F. In a state in which the abovementioned engaging part 12F is engaged with the opening part 11K, protrusions 11T which act as anchoring parts that are anchored with recesses 12H that act as anchored parts disposed in two locations in the circumferential direction on the outer surface of the abovementioned engaging part 12F, with these protrusions 11T being disposed in a state in which these parts protrude inward into the opening part 11K of the abovementioned base main body 11A, thus devising the system so that the handle part 12 cannot easily slip off of the base main body 11A. Furthermore, in Fig. 12, the recesses 12H and protrusions 11T respectively appear to be entities with surfaces on mutually opposite sides. Moreover, as is shown in Fig. 16, the anchoring force between the abovementioned anchoring parts and anchored parts is set so that in cases where the handle part 12 is pulled out in a state in which the sputum-collecting device 13 that has collected sputum is inserted into the collection sample container 14, the base part 11 remains "as is" in a state in which this base part is inserted into the interior of the collection sample container 14 in the state shown in the figures, and only the handle part 12 is separated from the base part 11.

The sputum-collecting device 14 shown in Figs. 14 and 15 comprises a container main body 14A which has substantially the same shape as the container main body 14A shown in Fig. 5, and a cap 14B consisting of an engaging part 14D that internally engages with the upper end portion inside wall surface 14H of the lower part 14a that constitutes the container main body 14A connected to the lower end of a substantially circular plate form grip part 14C. The symbol 15 indicated in the figures is the abovementioned removal piece 15, and is integrally formed on the lower end of the container main body 14A. Furthermore, a V-shaped peripheral groove 14X is formed in the bottom surface of the abovementioned upper part 14b, so that the removal piece 15 can easily be twisted off.

The V-shaped peripheral groove 14V used as a cut-away portion that allows the easy twisting off of the upper part 14a is formed in substantially the boundary area between the upper part 14a and lower part 14b of the abovementioned container main body 14A, and is devised so that the upper part 14a can easily be cut away. Here, a peripheral groove 14V is indicated; however, it would also be possible to form thin parts in a plurality of locations in the circumferential direction, as long as these parts can easily be twisted off.

Furthermore, a peripheral groove 14M for holding ring-form packing 21 is formed in the lower end of the abovementioned engaging part 14D, so that the leakage of the preservative liquid inside the container main body 14A to the outside is securely prevented.

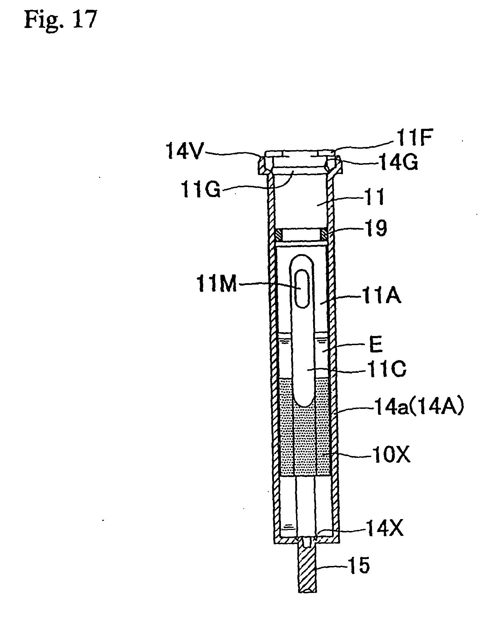

As is shown in Fig. 16, the collection sample container 14 into which the abovementioned sputum-collecting device 13 that has collected sputum has been inserted is placed in the envelope 6, and is sent by mail to a specified inspection organ; however, as is shown in Fig. 17, the collection sample container 14 in a state in which the upper part 14b has been twisted off along with the removal of the handle part 12 ma7 be placed in the envelope 6 and sent by mail, the collection sample container 14 in a state in which only the handle part 12 has been removed may be placed in the envelope 6 and sent by mail, or the collection sample container 14 in a state in which only the upper part 14b has been twisted off may be placed in the envelope 6 and sent by mail. Fig. 17 shows the collection sample container 14 in a state in which the handle part 12 has been removed and the upper part 14b has been twisted off in order to prevent collisions caused by the upper part which has a large width in the left-right width direction when centrifuging tubes are lined up in a tube rack after samples have been mounted in these centrifuging tubes.

### INDUSTIRAL APPLICABILTIY

In addition to the shipping of sputum-collecting tools that have collected sputum to inspection organs or the like by mail, these sputum-collecting tools can also be directly carried. Besides using various types of synthetic resins, the collection sample container of the sputum-collecting tool can be manufactured using any type of material such as a metal, wood, etc., as long as the material used does not easily undergo deformation or allow liquid leakage.

## Claims

1. A sputum-collecting tool comprising:
a sputum-collecting device in which a handle part is disposed on one end of a base part which has a sputum-collecting part that collects sputum attached to the other end; and
a collection sample container for accommodating this sputum-collecting device therein;
**characterized in that** grip faces that are positioned on both ends of said handle part in a thickness direction are formed substantially flat, and the left-right width dimension of said handle part is set greater than the left-right width dimension of said base part.

2. The sputum-collecting tool according to claim 1, wherein recesses or protrusions, or both, are formed in substantially the central portions of said grip faces.

3. The sputum-collecting tool according to claim 1, comprising a stopper on the base part end portion of said handle part which prevents the fingers grasping said handle part from moving toward the base part by contacting said fingers.

4. The sputum-collecting tool according to claim 1, wherein a fiber bundle formed by bundling a plurality of fibers for sucking up the sputum collected by said sputum-collecting part is disposed on said base part, and an intermediate portion of this fiber bundle is colored with a coloring agent that can be dispersed by the sputum.

5. The sputum-collecting tool according to claim 1, wherein said collection sample container comprises a container main body which is equipped at one end thereof with a removal piece that forms a projection protruding to the outside, and that can be cut away to allow removal of an internal accumulated liquid, and a freely detachable closing cap for closing off an opening part formed in the other end of this container main body.

6. The sputum-collecting tool according to claim 1 or claim 5, wherein a flange part for placing said collection sample container in a tightly closed state by engaging with the inner surface of said collection sample container when the insertion of said sputum-collecting device into said collection sample container is completed is disposed between the handle part and base part of said sputum-collecting device.

7. The sputum-collecting tool according to claim 1, claim 5 or claim 6, wherein at least a portion of the handle part of said sputum-collecting device is covered by the container main body of said collection sample container in a state in which the insertion of said sputum-collecting device into said collection sample container is completed.

8. The sputum-collecting tool according to claim 5, wherein a leg part for making said collection sample container self-stand is disposed on the removal piece of said collection sample container.

9. The sputum-collecting tool according to claim 1, wherein the handle part of said sputum-collecting device is disposed in the base part of said sputum-collecting device so that said handle part can be separated from said base part as said handle part is pulled out in order to remove said sputum-collecting device from said collection sample container in a state in which said sputum-collecting device is inserted into and accommodated in said collection sample container.

10. The sputum-collecting tool according to claim 1 or claim 9, wherein said collection sample container comprises a bottomed-cylindrical lower part which accommodates the base part of said sputum-collecting device, and an upper part which is integrally connected with the upper end portion of this lower part, and which is constructed with a size so as to cover at least a portion of the handle part of said sputum-collecting device from the outside, and a cut part is provided which allows said lower part and upper part to be cut apart in the connecting portion of said both parts.

11. The sputum-collecting tool according to any of claims 1 through 10, wherein said sputum-collecting part is made of polyurethane.

12. The sputum-collecting tool according to any of claims 1 through 10, wherein said sputum-collecting part is made of cellulose acetate.

13. The sputum-collecting tool according to any of claims 1 through 12, wherein the thickness of said collection sample container is set at 10 mm or less.

14. A sputum-collecting tool kit comprising:
a box form casing main body accommodating a sputum-collecting tool which comprises a sputum-collecting device in which a handle part is disposed on one end of a base part which has a sputum-collecting part that collects sputum attached to the other end, and a collection sample container for accommodating this sputum-collecting device therein, and which is **characterized in that** grip faces that are positioned on both ends of said handle part in a thickness direction are formed substantially flat, and the left-right width dimension of said handle part is set greater than the left-right width dimension of said base part; and
a cover body which is attached to said casing main body so that this cover body can be freely opened and closed by swinging, in order to close off the open side of said casing main body in which the sputum-collecting tool is accommodated.

15. The sputum-collecting tool kit according to claim 14, wherein a holding part for holding said collection sample container in an upright attitude is disposed in said casing main body.

16. The sputum-collecting tool kit according to claim 14, wherein a diagram explaining use is printed on the inside surface of said cover body, or a printed diagram explaining use is attached to the inside surface of said cover body.

17. The sputum-collecting tool kit according to claim 14 or claim 16, comprising an accommodating part that can accommodate a mailing envelope is disposed in said cover body.

18. The sputum-collecting tool kit according to claim 14, wherein the handle part of said sputum-collecting device is disposed in the base part of said sputum-collecting device so that this handle part can be separated from said base part as said handle part is pulled out in order to remove said sputum-collecting device from collection sample container in a state in which said sputum-collecting device is inserted into and accommodated in said collection sample container.

19. The sputum-collecting tool kit according to claim 14 or claim 18, wherein said collection sample container comprises a bottomed-cylindrical lower part which accommodates the base part of said sputum-collecting device, and an upper part which is integrally connected with the upper end portion of this lower part, and which is constructed with a size so as to cover at least a portion of the handle part of said sputum-collecting device from the outside, and a cut part is provided which allows said lower part and upper part to be cut apart in the connecting portion of said both parts.

20. The sputum-collecting tool kit according to any of claims 14 through 19, wherein said sputum-collecting part is made of polyurethane.

21. The sputum-collecting tool kit according to any of claims 14 through 19, wherein said sputum-collecting part is made of cellulose acetate.

22. The sputum-collecting tool kit according to any of claims 14 through 21, wherein the thickness of said collection sample container is set at 10 mm or less.
